# EUROPÄISCHE PATENTSCHRIFT

(11) **EP 2 440 114 B1**
(45) Veröffentlichungstag und Bekanntmachung des Hinweises auf die Patenterteilung: **20.04.2016**
(21) Anmeldenummer: 10722703.5
(22) Anmeldetag: 02.06.2010
(51) Int. Cl.: A61B 5/00

(54) **VORRICHTUNG ZUR TRANSKUTANEN, IN-VIVO MESSUNG DER KONZENTRATION ZUMINDEST EINES ANALYTEN IN EINEM LEBENDEN ORGANISMUS**
DEVICE FOR THE TRANSCUTANEOUS, IN VIVO MEASUREMENT OF THE CONCENTRATION OF AT LEAST ONE ANALYTE IN A LIVING ORGANISM
DISPOSITIF POUR LA MESURE TRANSCUTANÉE IN VIVO DE LA CONCENTRATION D'AU MOINS UN ANALYTE DANS UN ORGANISME VIVANT

(30) Priorität: 09.06.2009 AT 8912009
(43) Veröffentlichungstag der Anmeldung: 18.04.2012
(73) Patentinhaber: Joanneum Research Forschungsgesellschaft mbH, 8010 Graz (AT); Technische Universität Graz, 8010 Graz (AT)
(72) Erfinder: KÖHLER, Hans, A-8046 Graz (AT); KLIMANT, Ingo, A-8200 Labuch (AT)
(74) Vertreter: Dilg, Haeusler, Schindelmann Patentanwaltsgesellschaft mbH
(86) Internationale Anmeldenummer: PCT/EP2010/057732
(87) Internationale Veröffentlichungsnummer: WO 2010/142590

(56) Entgegenhaltungen:
- WO-A1-96/25089
- WO-A1-96/36275
- US-A- 5 246 867
- US-A- 6 032 059
- US-A1- 2006 263 839
- US-A1- 2008 269 723

## Beschreibung

Die Erfindung betrifft eine Vorrichtung zur transkutanen, in-vivo Messung der Konzentration zumindest eines Analyten in einem lebenden Organismus mit einem in den Organismus einbringbaren Träger und einem auf dem Träger immobilisierten Lumineszenzindikator, der auf eine Änderung der Konzentration des zu messenden Analyten mit einer Änderung zumindest einer optischen Eigenschaft reagiert, wobei der Lumineszenzindikator mit einer Quelle zur Bereitstellung der Anregungsstrahlung und einem Detektor zur Erfassung der Messstrahlung transkutan in Verbindung steht.

Im Folgenden wird unter dem Begriff Lumineszenzindikator nicht nur ein nach Anregung fluoreszierender oder phosphoreszierender Farbstoff verstanden, sondern auch eine Beschichtung, welche den Farbstoff in einem funktionellen Matrixmaterial aufnimmt.

Für Patienten, die die Konzentration eines bestimmten Analyten im Körper ständig überwachen müssen und dem Körper Medikamente zur Anpassung dieses Analyten an physiologische Werte zuführen müssen, ergibt sich ein beträchtlicher Aufwand.

So müssen beispielsweise Patienten mit Diabetes mehrmals täglich die Blutglukose messen, welche als Basis für eine Therapieentscheidung herangezogen wird. Dabei wird beispielsweise mit Hilfe einer Lanzette die Haut perforiert und das dabei austretende Blut auf einen Messstreifen als Teil eines Messsystems aufgebracht. Nach Vorliegen des Messwertes wird die Insulindosis errechnet, wobei das Insulin mit Hilfe einer Nadel in das Fettgewebe gespritzt wird. Dieser Mess/Injektions-Zyklus ist mit zwei Hautpenetrationen verbunden, die Schmerzen verursachen. Weiters wird das Insulin in einer "nicht physiologischen" Dosierung verabreicht. Um die gleichmäßige Dosierung zu optimieren, wurden Insulinpumpen entwickelt, welche das Insulin durch einen implantierbaren Katheter kontinuierlich abgeben. Neben dem Vorteil der kontinuierlichen Abgabe des Insulins, kann der Pumpenkatheter für längere Zeit im Gewebe verbleiben wodurch ein mehrmaliges Stechen entfällt und damit verbundene Unannehmlichkeiten vermieden werden können.

Aus der AT 408.182 B ist in diesem Zusammenhang ein Glukose-Sensor bekannt, bei welchem mit Hilfe einer Setznadel ein Katheter in das Gewebe eines lebenden Organismus eingebracht wird. Nach der Positionierung des Katheters im Gewebe wird die Setznadel zurückgezogen und durch einen rohrförmigen Träger mit einem elektrochemischen Sensor ersetzt. Der Sensor ist am äußeren Umfang des rohrförmigen Trägers im Bereich einer Wandöffnung des in das Gewebe eingeführten Katheters angeordnet, so dass ein Messkontakt zum umgebenden Gewebe hergestellt werden kann. Die elektrische Anschlussleitung des Sensors wird im Ringspalt zwischen rohrförmigem Träger und Katheter nach außen zu einer Auswerteeinheit geführt. Das Innenlumen des rohrförmigen Trägers, sowie der ringförmige Kanal zwischen dem rohrförmigen Träger und dem Katheter ist jeweils mit einer Spritzenpumpe verbunden, mit deren Hilfe Flüssigkeiten in das Gewebe eingebracht werden können. Bei einer Insulingabe ist somit mit dieser Vorrichtung für Messung und Applikation nur eine Hautpenetration notwendig.

Aus der WO 96/36275 ist ein Verfahren und eine Vorrichtung zur transkutanen Messung eines Analyten in lebendem Gewebe bekannt, bei welchem ein in einem Trägergewebe immobilisierter Fluoreszenzindikator im Gewebe implantiert wird. Als Träger wird eine planare oder zylindrische Membran, beispielsweise eine Zellulosemembran, verwendet, welche für Glukose permeabel ist. Die Membran enthält einen gewebeverträglichen Fluoreszenzindikator, der auf die Änderung der Glukosekonzentration mit einer Änderung seiner Fluoreszenzabklingzeit oder mit einer Frequenzverschiebung reagiert. Die Anregungsstrahlung wird von einer außenliegenden Lichtquelle durch das Gewebe eingestrahlt, die entstehende Messstrahlung gelangt durch das Gewebe und die Haut in den außenliegenden Detektor dessen Signale einer Auswerte- und Anzeigeeinheit zugeführt werden. Die Verabreichung eines Medikamentes ist allerdings durch die implantierte Sensorvorrichtung weder vorgesehen noch möglich, so dass beispielsweise für eine Insulingabe nach wie vor eine zusätzliche Hautpenetration notwendig wäre.

Weiters sind auch eine Reihe von Anwendungen bekannt (siehe z.B. US 2009/0088615 A1), bei welchen in-vivo Glukosemessungen mit einem in das Gewebe einführbaren optischen Lichtleiter durchgeführt werden, wobei unterschiedliche Anwendungen beschrieben werden, mit welchen die Messstrahlung von der Anregungsstrahlung getrennt werden kann.

Weiters ist aus der WO 2006/102412 A2 eine Vorrichtung bekannt, welche eine Insulinpumpe und eine Einrichtung zur Glukosemessung aufweist. Es werden Ausführungsvarianten beschrieben, bei welchen auf einer Infusionskanüle für ein Medikament elektrochemische Messeinrichtungen, bestehend aus Arbeits-, Referenz- und Gegenelektroden befestigt sind, um nach dem Einbringen der Kanüle ins Gewebe eine Analytkonzentration zu messen. Gemäß einer weiteren Ausführungsvariante wird ein elektrochemischer Sensor mit mehreren Elektroden möglichst klein ausgeführt und um den äußeren Umfang des in das Gewebe einführbaren Katheters gewickelt. Alle diese Ausführungsvarianten sind technisch sehr aufwändig, vergrößern den Umfang der Gewebekanüle und beeinträchtigen die Güte der Oberfläche der Kanüle, wodurch das Einführen in das Gewebe erschwert wird.

US 2008/269723 A1 offenbart ein Glukose-Sensorsystem, eine Steuervorrichtung und ein Insulinverabreichungssystem. Das System weist einen Sensor mit einem Abfühlende auf, welches freigelegte Elektroden aufweist und durch eine Haut in ein subkutanes Gewebe eines Körpers eines Benutzers eingeführt wird.

US 2006/0263839 A1 offenbart eine Ausführungsform einer Vorrichtung, bei welcher eine Röhre, wie beispielsweise ein Katheter, mit einem Sensormodul verbunden ist. Auf der Außenseite der Röhre kann eine Anzeigeelektrode angeordnet sein, die mit dem Sensormodul über eine leitfähige Bahn elektrisch verbunden ist. Auf der Außenseite der Röhre kann ferner eine Referenzelektrode angeordnet sein, die mit dem Sensormodul über eine leitfähige Bahn elektrisch verbunden ist.

Aufgabe der Erfindung ist es, ausgehend von den bekannten Lösungen für die Messung eines Analyten und die Abgabe eines Medikamentes in den Organismus, Verbesserungen vorzuschlagen, die eine atraumatische Behandlung bei geringen Kosten der Vorrichtung zulassen.

Diese Aufgabe wird erfindungsgemäß dadurch gelöst, dass der Lumineszenzindikator am äußeren Umfang eines Katheters immobilisiert ist, der zur Abgabe eines flüssigen Mediums, beispielsweise eines Medikamentes, in den Organismus oder zum Absaugen einer Körperflüssigkeit dient. Als Katheter wird eine dünne Einmal-Nadel, beispielsweise aus Stahl oder Kunststoff, eingesetzt, wie sie z.B. in der Insulintherapie verwendet wird.

Die Erfindung kombiniert z.B. die Applikation eines Medikamentes mit der Messung einer Analytkonzentration derart, dass die Außenseite beispielsweise eines Gewebekatheters mit einem lumineszenzoptischen Indikator beschichtet ist. Die analytspezifische Antwort des Lumineszenzindikators wird mit Hilfe eines Detektors an der Hautoberfläche erfasst und die Analytkonzentration berechnet. In vorteilhafter Weise erfolgt die Anregung des Luminophors und die Messung der Lumineszenzstrahlung nicht-invasiv als elektromagnetische Strahlung durch das Gewebe, wobei keinerlei Anschlüsse wie bei elektrochemischen Sensoren oder Lichtleiter, wie bei optischen Sensoren, in das Gewebe eingebracht werden müssen. Vorteile bestehen auch gegenüber der zum Stand der Technik zitierten WO 96/36275, bei welcher der Fluoreszenzindikator samt Trägermatrix implantiert wird. Ein zusätzlicher Aufwand ist hier beispielsweise am Ende der Nutzungsdauer mit der Entfernung des implantierten Sensors verknüpft.

Die erfindungsgemäße Vorrichtung kann auch zum Absaugen einer Körperflüssigkeit verwendet werden. Beispielsweise kann Wundsekret aus einer Operationswunde abgesaugt werden und gleichzeitig an der Außenseite des Katheters die Sauerstoffkonzentration als Indikator der lokalen Versorgung des Gewebes gemessen werden. Dadurch kann ein indirekter Rückschluss auf den Wündheilungsprozess gewonnen werden.

Durch das Immobilisieren des Lumineszenzindikators direkt auf den Infusionskatheter wird dieser automatisch mit dem Entfernen des Katheters aus dem Körper entfernt. Neben dem gemeinsamen, transkutanen Zugang für Messung und Medikamentenapplikation oder Gewinnung von Körperflüssigkeit besteht ein weiterer Vorteil darin, dass das System äußerst kostengünstig hergestellt werden kann, weil lediglich der Katheter mit der Beschichtung ausgewechselt werden muss, die übrige Einheit mit Elektronik, Optik, etc. aber mehrfach verwendbar ist.

Erfindungsgemäß kann der Katheter am äußeren Umfang eine beispielsweise ringförmige Vertiefung oder Anätzung bzw. Aufrauung zur Aufnahme des vorzugsweise in einer Trägerschicht vorliegenden Lumineszenzindikators aufweisen.

Es ist auch möglich, den Lumineszenzindikator in zumindest einem Segment des Katheters durch physikalische Fixierung oder chemische Bindung zu immobilisieren. Das mit dem Lumineszenzindikator beschichtete Segment steht in direktem Kontakt mit der Körperflüssigkeit, wobei die Beschichtung des Katheters auch partiell (z.B. einseitig) erfolgen kann. Das Segment umfasst eine größere Fläche, um punktuelle Inhomogenitäten der zu messenden Analytkonzentration im Organismus auszugleichen.

Eine besonders einfache Anwendung der erfindungsgemäßen Vorrichtung ist durch eine Ausführungsvariante gewährleistet, bei welcher der Katheter nach außen vorstehend in einem Basiselement eines Gehäuses angeordnet ist, wobei das Basiselement unter Einführung des Katheters auf die Oberfläche des Organismus aufsetzbar ist.

Weiters ist erfindungsgemäß vorgesehen, dass im Gehäuse eine Elektronikeinheit vorliegt, welche die Bereitstellung der Anregungsstrahlung und die Erfassung der Messstrahlung steuert, die Analytkonzentration berechnet und/oder in Abhängigkeit der Analytkonzentration eine Medikamentendosis festlegt. Weiters kann die Elektronikeinheit die Dosiereinheit für das Medikament steuern und die zuvor festgelegte Medikamentendosis bevorzugt automatisch in den Organismus applizieren.

Die Erfindung wird im Folgenden anhand von schematischen Zeichnungen näher erläutert. Es zeigen:
- Fig. 1: eine erste Ausführungsvariante der Erfindung in einer teilweisen Schnittdarstellung,
- Fig. 2: eine Schnittdarstellung gemäß Linie II-II in Fig. 1,
- Fig. 3: eine vereinfachte Ausführungsvariante der erfindungsgemäßen Vorrichtung in einer Schnittdarstellung, sowie
- Fig. 4: eine schematische Darstellung der einzelnen funktionellen Komponenten der erfindungsgemäßen Vorrichtung.

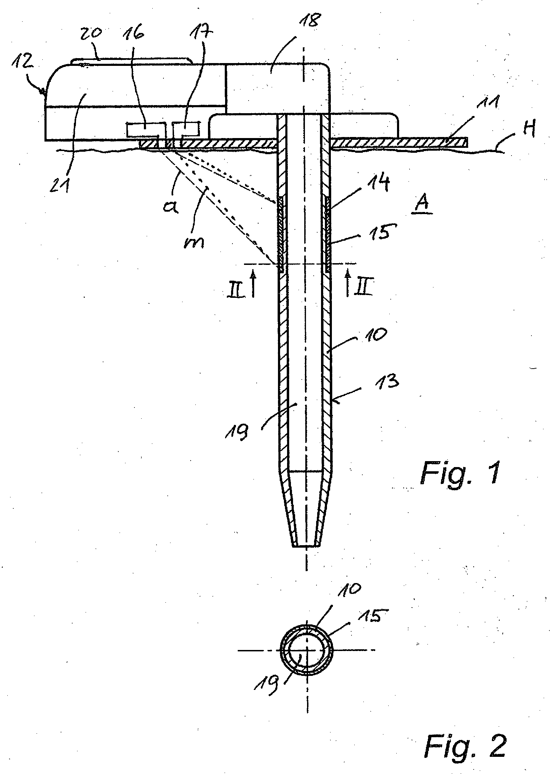

Die in den Fig. 1 und 2 dargestellte erfindungsgemäße Vorrichtung zur transkutanen, in-vivo Messung der Konzentration eines Analyten (z.B. Glukose) im Gewebe eines Organismus A mit einer Hautoberfläche H weist einen in das Gewebe einstechbaren Katheter 10 auf, der nach außen vorstehend in einem Basiselement 11 eines Gehäuses 12 der Vorrichtung angeordnet ist. Der Katheter 10 weist am äußeren Umfang 13 eine ringförmige Vertiefung oder Anätzung 14 zur Aufnahme des vorzugsweise in einer Trägerschicht vorliegenden Lumineszenzindikators 15 auf.

Das Gehäuse 12 der Vorrichtung nimmt eine Quelle 16 zur Bereitstellung der Anregungsstrahlung a und einen Detektor 17 zur Erfassung der Messstrahlung m auf. Um störende Absorptionen im Gewebe zu vermeiden, sollte die Anregungs- und Messstrahlung in einem Bereich von ca. 600 bis 1100 nm liegen.

Im dargestellten Beispiel ist im Gehäuse eine Dosiereinheit 18 zur Abgabe eines Medikamentes (z.B. Insulin) vorgesehen, die mit dem Lumen 19 der Kanüle 10 in Verbindung steht.

Durch die Immobilisierung des Lumineszenzindikators 15 in einer ringförmigen Vertiefung 14 der Kanüle 10 kann eine glatte, ebene Oberfläche der Kanüle 10 beibehalten werden, ohne den äußeren Umfang 13 vergrößern zu müssen.

Im Gehäuse 12 der Vorrichtung ist weiters eine Elektronikeinheit 21 angeordnet, welche in Abhängigkeit der vom Lumineszenzindikator 15 gemessenen Analytkonzentration eine Medikamentendosis festlegt, die vorzugsweise automatisch in den Organismus A appliziert werden kann. Die Elektronikeinheit 21 dient auch zur Steuerung des Messvorgangs und zur Berechnung der Analytkonzentration.

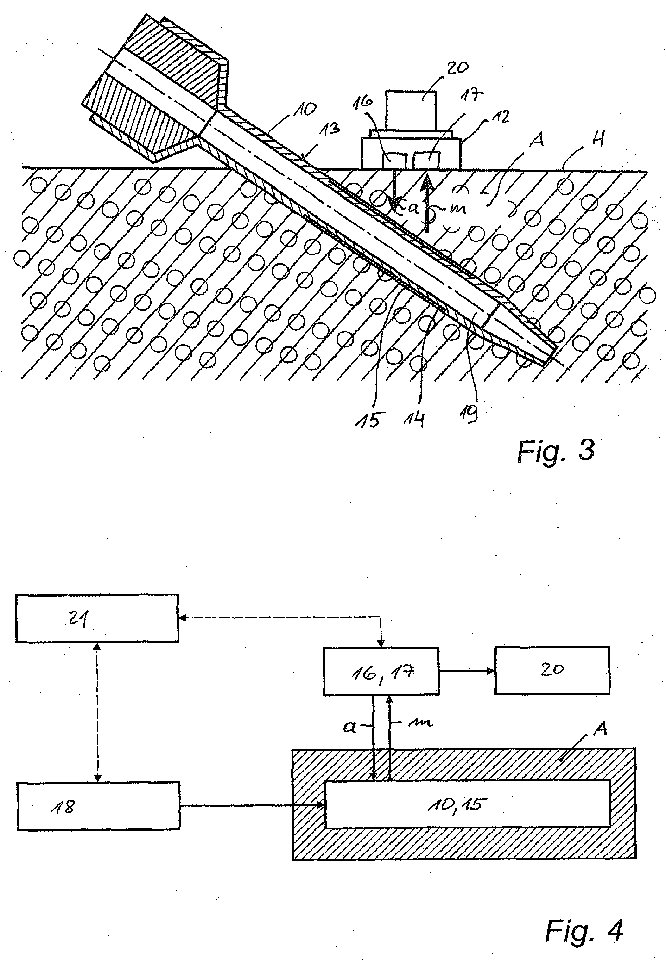

Die Ausführungsvariante gemäß Fig. 3 zeigt eine erfindungsgemäße Vorrichtung, bei welcher der Katheter 10 zur Abgabe eines Medikamentes in den Organismus A und das Gehäuse 12, welches die Anregungsquelle 16 und den Detektor 17 aufnimmt, separate Einheiten darstellen. Bei dieser Ausführungsvariante muss das Gehäuse 12 in der Nähe des eingeführten Katheters 10 auf die Hautoberfläche H des Organismus A aufgesetzt werden, um den Lumineszenzindikator 15 durch das Gewebe hindurch anzuregen und die Messstrahlung m zu erfassen. Es kann beispielsweise eine herkömmliche Insulinpumpe zum Einsatz kommen, wobei die Messwerte von Hand oder automatisch (per Kabel oder Funksignal) von der Messeinheit zur Insulinpumpe übertragen werden.

Fig. 4 zeigt in schematischer Darstellung die einzelnen funktionellen Komponenten der erfindungsgemäßen Vorrichtung. Der in den Organismus A eingeführte Katheter 10 mit dem Lumineszenzindikator 15 steht lediglich über die Anregungs- und Messstrahlung a, m mit der außen angeordneten Einheit mit Quelle 16 und Detektor 17 in Verbindung. Eine von der Elektronikeinheit 21 gesteuerte Dosiereinrichtung 18 appliziert die errechnete Medikamentendosis über den Katheter 10 in das Gewebe. Alle Messwerte und Dosisangaben können an einer Anzeigeeinheit 20 dargelegt werden.

Die erfindungsgemäße Vorrichtung eignet sich bestens zur Bestimmung der Gewebsglukose und Verabreichung der benötigten Insulinmengen, kann aber auch für Messungen in anderen Körperflüssigkeiten wie Blut, Lymphe, Hirnflüssigkeit o.a. herangezogen werden.

Neben Glukose kann aber auch Laktat, Sauerstoff, pH, Elektrolyte oder eine andere endogene oder eine exogen zugeführte Substanz gemessen werden.

Als Medikamente können Insulin oder Insulinanaloga, aber auch Glucagon, GLP (Glucagon like Pepdite), Wachstumshormone etc. zugeführt werden.

Die erfindungsgemäße Vorrichtung kann sowohl in der Humanmedizin als auch in der Tiermedizin eingesetzt werden.

## Patentansprüche

1. Vorrichtung zur transkutanen, in-vivo Messung der Konzentration zumindest eines Analyten in einem lebenden Organismus (A) mit einem in den Organismus einbringbaren Träger und einem auf dem Träger immobilisierten Lumineszenzindikator (15), der auf eine Änderung der Konzentration des zu messenden Analyten mit einer Änderung zumindest einer optischen Eigenschaft reagiert, wobei der Lumineszenzindikator (15) mit einer Quelle (16) zur Bereitstellung der Anregungsstrahlung (a) und einem Detektor (17) zur Erfassung der Messstrahlung (m) transkutan in Verbindung steht,
wobei
der Lumineszenzindikator (15) am äußeren Umfang eines Katheters (10) immobilisiert ist, der zur Abgabe eines flüssigen Mediums, beispielsweise eines Medikamentes, in den Organismus (A) oder zum Absaugen einer Körperflüssigkeit dient,
der Katheter (10) nach außen vorstehend in einem Basiselement (11) eines Gehäuses (12) angeordnet ist, wobei das Basiselement (11) unter Einführung des Katheters (10) auf die Oberfläche (H) des Organismus (A) aufsetzbar ist, und
das Gehäuse (12) die Quelle (16) zur Bereitstellung der Anregungsstrahlung (a) und den Detektor (17) zur Erfassung der Messstrahlung (m) aufnimmt.

2. Vorrichtung nach Anspruch 1, **dadurch gekennzeichnet, dass** der Lumineszenzindikator (15) in zumindest einem Segment des Katheters (10) durch physikalische Fixierung oder chemische Bindung immobilisiert ist.

3. Vorrichtung nach Anspruch 1, **dadurch gekennzeichnet, dass** der Katheter (10) am äußeren Umfang (13) eine Vertiefung (14) oder Anätzung zur Aufnahme des vorzugsweise in einer Trägerschicht vorliegenden Lumineszenzindikators (15) aufweist.

4. Vorrichtung nach einem der Ansprüche 1 bis 3, **dadurch gekennzeichnet, dass** die Vorrichtung eine Dosiereinheit (18) für ein Medikament aufweist, die mit dem Katheter (10) in Verbindung steht, wobei die Medikamentendosis vorzugsweise in Abhängigkeit der vom Lumineszenzindikator (15) gemessenen Analytkonzentration einstellbar ist.

5. Vorrichtung nach Anspruch 4, **dadurch gekennzeichnet, dass** das Gehäuse (12) die Dosiereinheit (18) zur Abgabe eines Medikamentes aufnimmt.

6. Vorrichtung nach einem der Ansprüche 1 bis 5, **dadurch gekennzeichnet, dass** im Gehäuse (12) eine Elektronikeinheit (21) vorliegt, welche die Bereitstellung der Anregungsstrahlung (a) und die Erfassung der Messstrahlung (m) steuert, die Analytkonzentration berechnet und in Abhängigkeit der Analytkonzentration eine Medikamentendosis festlegt.

7. Vorrichtung nach Anspruch 6, welche ferner die Merkmale von Anspruch 5 aufweist, **dadurch gekennzeichnet, dass** die Elektronikeinheit (21) die Dosiereinheit (18) für das Medikament steuert und die festgelegte Medikamentendosis bevorzugt automatisch in den Organismus (A) appliziert.

8. Vorrichtung nach einem der Ansprüche 1 bis 6, **dadurch gekennzeichnet, dass** der zu messende Analyt ein endogener Metabolit, beispielsweise Glukose oder Laktat, oder eine exogen zugeführte Substanz ist.

## Claims

1. Device for transcutaneous in vivo measurement of the concentration of at least one analyte in a living organism (A), with a carrier insertable into the organism and, immobilized on the carrier, a luminescence indicator (15) which reacts on a change of the concentration of the analyte to be measured with a change of at least one optical characteristic, wherein the luminescence indicator (15) is transcutaneously connected with a source (16) for providing the excitation radiation (a) and a detector (17) for detecting the measurement radiation (m), wherein
the luminescence indicator (15) is immobilized at an outer periphery of a catheter (10) serving for delivery of a liquid medium, for example a medicament, into the organism (A) or for sucking up a body fluid,
the catheter (10) is positioned outwardly protruding in a base element (11) of a housing (12), wherein the base element (11) is putable onto the surface (H) of the organism (A) under insertion of the catheter (10), and
the housing (12) houses the source (16) for providing the excitation radiation (a) and the detector (17) for detecting the measurement radiation (m).

2. Device according to claim 1, **characterized in that** the luminescence indicator (15) is immobilized in at least one segment of the catheter (10) by physical fixation or chemical bonding.

3. Device according to claim 1, **characterized in that** the catheter (10) has at the outer periphery (13) a recess (14) or an etching for reception of the luminescence indicator (15) which is existent preferably in a carrier layer.

4. Device according to one of the claims 1 to 3, **characterized in that** the device has a dosage unit (18) for a medicament connected with the catheter (10), wherein the medicament dose is adjustable preferably depending on the analyte concentration measured by the luminescence indicator (15).

5. Device according to claim 4, **characterized in that** the housing (12) houses the dosage unit (18) for delivery of a medicament.

6. Device according to one of the claims 1 to 5, **characterized in that** there is existent in the housing (12) an electronic unit (21) which controls the provision of the excitation radiation (a) and the detection of the measurement radiation (m), calculates the analyte concentration, and determines, depending on the analyte concentration, a medicament dose.

7. Device according to claim 6, which further comprises the features of claim 5, **characterized in that** the electronic unit (21) controls the dosage unit (18) for the medicament and applies the determined medicament dose preferably automatically into the organism (A).

8. Device according to one of the claims 1 to 6, **characterized in that** the analyte to be measured is an endogenous metabolite, for example glucose or lactate, or an exogenously administered substance.

## Revendications

1. Dispositif servant à la mesure in vivo transcutanée de la concentration au moins d'un analyte dans un organisme (A) vivant, comprenant un support pouvant être introduit dans l'organisme et un indicateur de luminescence (15) immobilisé sur le support, lequel réagit à une modification de la concentration de l'analyte à mesurer avec une modification au moins d'une propriété optique, l'indicateur de luminescence (15) étant relié par voie transcutanée à une source (16) servant à mettre à disposition le rayonnement d'excitation (a) et à un détecteur (17) servant à détecter le rayonnement de mesure (m),
**caractérisé en ce que**
l'indicateur de luminescence (15) est immobilisé au niveau de la périphérie extérieure d'un cathéter (10), qui sert à distribuer un milieu liquide, par exemple un médicament, dans l'organisme (A) ou à évacuer par aspiration un liquide corporel,
le cathéter (10) est disposé de manière à faire saillie vers l'extérieur dans un élément de base (11) d'un boîtier (12), l'élément de base (11) pouvant être posé en introduisant le cathéter (10) sur la surface (H) de l'organisme (A), et
le boîtier (12) reçoit la source (16) servant à mettre à disposition le rayonnement d'excitation (a) et le détecteur (17) servant à détecter le rayonnement de mesure (m).

2. Dispositif selon la revendication 1, **caractérisé en ce que** l'indicateur de luminescence (15) est immobilisé dans au moins un segment du cathéter (10) par une fixation physique ou par une liaison chimique.

3. Dispositif selon la revendication 1, **caractérisé en ce que** le cathéter (10) présente, au niveau de la périphérie (13) extérieure, un renfoncement (14) ou une trace d'attaque servant à recevoir l'indicateur de luminescence (15) présent de préférence dans une couche de support.

4. Dispositif selon l'une quelconque des revendications 1 à 3, **caractérisé en ce que** le dispositif présente une unité de dosage (18) pour un médicament, qui est reliée au cathéter (10), la dose de médicament pouvant être réglée de préférence en fonction de la concentration d'analyte mesurée par l'indicateur de luminescence (15).

5. Dispositif selon la revendication 4, **caractérisé en ce que** le boîtier (12) reçoit une unité de dosage (18) servant à distribuer un médicament.

6. Dispositif selon l'une quelconque des revendications 1 à 5, **caractérisé en ce qu'**est présente dans le boîtier (12) une unité électronique (21), qui commande la mise à disposition du rayonnement d'excitation (a) et la détection du rayonnement de mesure (m), qui calcule la concentration d'analyte et qui détermine une dose de médicament en fonction de la concentration d'analyte.

7. Dispositif selon la revendication 6, qui présente en outre les caractéristiques de la revendication 5, **caractérisé en ce que** l'unité électronique (21) commande l'unité de dosage (18) pour le médicament et applique la dose de médicament déterminée de manière préférée automatiquement dans l'organisme (A).

8. Dispositif selon l'une quelconque des revendications 1 à 6, **caractérisé en ce que** l'analyte à mesurer est un métabolite endogène, par exemple du glucose ou du lactate, ou une substance amenée de manière exogène.
